Europäisches Patentamt

European Patent Office

Office européen des brevets

(11) Publication number: **0 513 778 A2**

## EUROPEAN PATENT APPLICATION

(21) Application number: **92108134.5**

(22) Date of filing: **14.05.92**

(51) Int. Cl.5: **C12P 21/08**, A61K 39/395

(30) Priority: **17.05.91 JP 112874/91**
**13.03.92 JP 55025/92**

(43) Date of publication of application:
**19.11.92 Bulletin 92/47**

(84) Designated Contracting States:
**AT BE CH DE DK ES FR GB GR IT LI LU NL PT SE**

(71) Applicant: **TAKEDA CHEMICAL INDUSTRIES, LTD.**
**1-1, Doshomachi 4-chome**
**Chuo-ku, Osaka 541(JP)**

(72) Inventor: **Iwasa, Susumo**
**21-2, Ohsumigaoka 1-chome, Tanabe-cho**
**Tsuzuki-gun, Kyoto 610-03(JP)**
Inventor: **Kurokawa, Tomofumi**
**91-18, Daiwahigashi 1-chome**
**Kawanishi, Hyogo 666-01(JP)**
Inventor: **Watanabe, Akiko**
**2-502, 17 Kitaoogi 4-chome, Higashinada-ku**
**Kobe, Hyogo 658(JP)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) **Hybrid monoclonal antibodies and compositions containing them.**

(57) The present invention relates to a bispecific antibody having specificity for both a thrombus and a substance having thrombolytic activity, which contains a variable region and lacks heavy chain constant region domains 2 and 3, and a thrombolytic agent comprising said antibody and a thrombolytic substance immunologically bound thereto, whereby its side effects are extremely reduced.

EP 0 513 778 A2

FIELD OF THE INVENTION

The present invention relates to a bispecific hybrid monoclonal antibody containing a variable region and lacking heavy chain constant region domains, and more particularly to a bispecific monoclonal antibody (hereinafter occasionally referred to as MoAb) specific for both a thrombus and a substance having thrombolytic activity, which contains a variable region and lacks heavy chain constant region domains 2 and 3 (hereinafter occasionally referred to as $CH_2$ and $CH_3$, respectively).

In another aspect, the present invention is further directed to a thrombolytic agent comprising the above-mentioned bispecific MoAb and a substance having thrombolytic activity immunologically bound thereto.

BACKGROUND OF THE INVENTION

Thrombolytic therapeutics have been widely used for treatment of patients with thrombotic diseases such as cardiac infarction, arterial embolism and cerebral infarction, and streptokinase (hereinafter occasionally referred to as SK) and urokinase (hereinafter occasionally referred to as UK) have been clinically employed. In recent years, tissue plasminogen activator (hereinafter occasionally referred to as TPA) and prourokinase (hereinafter occasionally referred to as ProUK) have appeared which are said to have selectivity for thrombi resulting in the reduced side effect of the tendency to cause bleeding, and are replacing SK and UK described above. Furthermore, as more effective thrombolytic agents excellent in thrombus selectivity, modified TPA and a hybrid protein of UK and TPA have been developed. On the other hand, thrombolytic agents utilizing antibody targeting have also appeared [see C. Bode et al., Science, 229, 765 (1985); M. S. Runge et al., Proc. Natl. Acad. Sci. USA, 84, 7659 (1987); T. Kurokawa et al., Bio/Technology, 7, 1163 (1989); European Patent Unexamined Publication No. 0363712; and European Patent Unexamined Publication No. 0450479]. For example, utilizing antibodies having high affinity for fibrinogen forming thrombi or for activated platelets, thrombolytic agents have been prepared which exhibit the efficiency several times to several tens of times higher than substances having thrombolytic activity alone. In particular, bispecific MoAbs which can be immunologically bound to both thrombi and substances having thrombolytic activity provide highly efficient thrombolytic agents not accompanied by a reduction in antibody activity and in thrombolytic activity.

Such antibody targeted thrombolytic agents not only enhance thrombolytic capability of the substances having thrombolytic activity, but also prolong their blood half-life. At the same time, however, it is unavoidable to some extent that the side effects [fibrinogenolysis activity and $\alpha_2$-antiplasmin (hereinafter occasionally referred to $\alpha_2$-AP) consuming activity) of the substances having thrombolytic activity are increased.

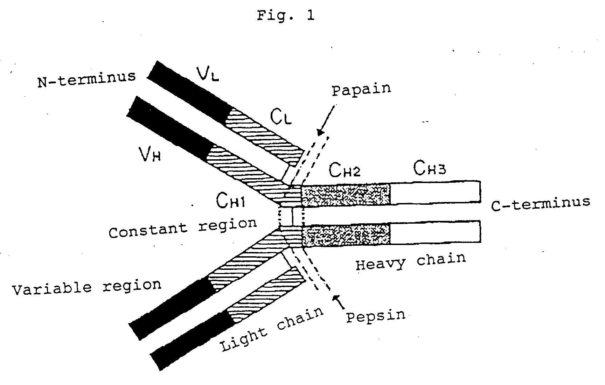

An antibody molecule has, as shown in Fig. 1, variable (V) regions related to binding to an antigen and constant (C) regions not related to antigen binding, but related to other biological functions such as binding to a complement and binding to an antibody receptor. When only the function related to antigen binding is required, molecules composed of constant regions alone are adequate for such a purpose. Such molecules have recently been prepared by recombinant technology. An Fab fragment or an Fab' fragment lacking heavy (H) chain constant region domains 2 and 3 ($CH_2$ and $CH_3$) has been prepared for a long time by the method of restrictedly partially hydrolyzing an antibody molecule with an enzyme such as papain or pepsin to cleave a peptide at the position shown in Fig. 1.

SUMMARY OF THE INVENTION

In order to solve the problem of increases in the side effects observed in the above-mentioned antibody targeted thrombolytic agents, the present inventors conducted various studies. As a result, the present inventors discovered that the use of a bispecific lower molecular weight antibody containing a variable region used for targeting to a thrombus can unexpectedly reduce its side effects while carrying or enhancing the thrombolytic activity enhancing effect of the original antibody molecule.

Namely, the present invention provides a bispecific antibody specific for both a thrombus and a substance having thrombolytic activity, which contains a variable region and lacks $CH_2$ and $CH_3$.

The present invention further provides a thrombolytic agent comprising the above-mentioned bispecific antibody and a substance having thrombolytic activity immunologically bound thereto.

BRIEF DESCRIPTION OF THE DRAWINGS

Fig. 1 is a schematic representation showing a structure of an antibody molecule;

Fig. 2 is a graph showing the results of bispecific antibody activity measured by an enzyme immunoassay (hereinafter occasionally referred to as EIA) described in Reference Example 7 for anti-UK-anti-fibrin bispecific MoAb FU 1-74 (●) and an F(ab')$_2$ fraction thereof (○) described in Example 1 [see Example 1-(3)];

Fig. 3 is a graph showing the results obtained by an in vitro plasma clot lysis assay described in Reference Example 1 for anti-UK-anti-fibrin bispecific MoAb FU 1-74 (closed) and the F(ab')$_2$ fraction thereof (shaded) described in Example 1 (see Test Example 1);

Fig. 4 is graphs showing the thrombolytic capability obtained when anti-UK-anti-fibrin bispecific MoAb FU 1-74 (○) and the F(ab')$_2$ fraction thereof (△) are given together with ProUK, comparing with that when ProUK alone (●) is given (see Test Example 2);

Fig. 5 is a graph showing the results of bispecific antibody activity measured by the EIA described in Reference Example 7 for anti-UK-anti-fibrin bispecific MoAb FU 1-74 (●) described in Example 1-(1) and an F(ab')$_2$ fraction (○) described in Example 4-(5) [see Example 4-(6)];

Fig. 6 is a graph showing the results of antibody response obtained when anti-UK-anti-fibrin bispecific MoAb FU 1-74 (●) described in Example 1-(1) and the F(ab')$_2$ fraction (○) described in Example 1-(2) are each intravenously given to monkeys (see Example 5); and

Fig. 7 is a graph showing the results of bispecific antibody activity measured by an EIA described in Reference Example 8 for an F(ab')$_2$ fraction (○) of an anti-UK-anti-activated platelet bispecific MoAb described in Example 6-(3) (see Example 6).

## DESCRIPTION OF THE PREFERRED EMBODIMENTS

In the preparation of anti-thrombus antibody-producing hybridomas such as anti-fibrin antibody-producing hybridomas used in the present invention, any hybridomas may be used as long as they produce MoAbs which are specific for fibrins and do not substantially bind to fibrinogen. For example, such fibrin specific antibodies are prepared by using as immunogens α-chain N-terminal fragment peptides or β-chain N-terminal fragment peptides of fibrins produced by decomposition of fibrinogen [K. Y. Hui et al., Science, 222, 1129 (1983); and Japanese Patent Unexamined Publication No. 63-93800/1988]. Any fibrins may be used as long as they are mammalian. Preferred examples thereof include human fibrin. A peptide corresponding to the β-chain N-terminal portion of human fibrin is used among others. Carrier proteins are bound thereto, and animals (such as rabbits, rats, mice and guinea pigs) are immunized therewith to obtain antibody-producing cells. Next, these antibody-producing cells recovered from the immunized animals, such as spleen cells and lymphatic node cells, are fused, and the antibody-producing cells which do not substantially react with fibrinogen and specifically bind to the fibrins are screened from the resulting hybridomas. As the above-mentioned β-chain N-terminal peptide of human fibrin, a peptide having the following amino acid sequence is particularly preferably used:

H-Gly-His-Arg-Pro-Leu-Asp-Lys-R-Cys-OH (SEQ ID No:1)

wherein R represents a peptide indicated by Lys-Arg-Glu-Glu or a portion thereof. Cys, the C-terminus, is used as a linker portion for chemical binding to the carrier protein. Namely, it is possible to chemically bind the above-mentioned peptide to the carrier protein through an SH group of Cys, the C-terminus of the peptide, by previously maleimidating the carrier protein with N-(γ-maleimidobutylyloxysuccimide (hereinafter occasionally referred to as GMBS) or dithiopyridylating the carrier protein with N-succimidyl-3-(2-pyridyldithio)propionate (hereinafter referred to as SPDP).

In the preparation of anti-activated platelet MoAb-producing hybridomas, any hybridomas may be used as long as they produce MoAbs which are specific for activated platelets and do not substantially bind to resting platelets. For example, such activated platelet specific MoAbs are prepared by using as immunogens platelets (preferably human platelets) activated with thrombin [C. L. Berman et al., J. Clin. Invest. 78, 130 (1986); and N. Akamatsu et al., Thromb. Haemostasis, 62, 250 (1989)]. Any platelets may be used as long as they are mammalian. Preferred examples thereof include human platelets. Animals such as rabbits, rats, mice and guinea pigs are immunized with the activated platelets to obtain antibody-producing cells. Next, these antibody-producing cells recovered from the immunized animals, such as spleen cells and lymphatic node cells, are fused with myeloma cells. The antibody-producing cells which do not substantially react with the resting platelets and specifically bind to the activated platelets are screened from the resulting hybridomas, whereby the desired anti-activated platelet MoAb-producing hybridoma cells can be obtained.

As the substances having thrombolytic activity (hereinafter occasionally referred to as thrombolytic substance), any substances may be used as long as they are proteins having thrombolytic activity or substances promoting thrombolytic activity. Examples thereof include proteases, precursors thereof and thrombolysis-promoting substances (for example, TPA, UK, ProUK, trypsin, plasmin, protein C and Protein S). In particular, proteases, especially plasminogen activator, are preferably used, and more preferably, TPA, UK and ProUK are used. TPA may be single-chain or two-chain types, and UK may also be single-chain or two-chain types [E. Haber et al., Science, 243, 51 (1989)]. Furthermore, low molecular weight UK may be used. However, ProUK is more preferably used among others. In the preparation of anti-thrombolytic substance MoAb-producing hybridomas, animals are immunized with the above-mentioned proteins according to methods known in the art, and the resulting antibody-producing cells are fused with myeloma cells and myeloma-like cells. In particular, in the preparation of anti-UK antibody-producing hybridomas, it is convenient to use antibody-producing cells obtained by immunizing animals with low molecular weight UK. The immunization of the animals and the fusion of the resulting antibody-producing cells with the myeloma cells to obtain the antibody-producing hybridoma cells may be carried out similarly with the preparation of the anti-fibrin antibody-producing hybridoma cells or the anti-activated platelet antibody-producing hybridoma cells.

Examples of the animals for immunization include rabbits, rats, mice and guinea pigs. When the MoAbs are prepared, mice are preferably used. The inoculation is performed according to a standard method. For example, when the antibody specific for the activated platelets is prepared, $10^8$ to $10^{10}$, preferably $0.5 \times 10^9$ to $2 \times 10^9$ washed human platelets each time suspended in physiological saline, Hepes buffer or phosphate buffered saline (hereinafter occasionally referred to as PBS) are activated with thrombin, and then inoculated into mice intraperitoneally, 3 to 8 times every 10 to 14 days. When the antibody specific for human fibrin or the thrombolytic substances is prepared, 1 to 100 μg each time, preferably 10 to 25 μg each time of the antigen protein emulsified with an equal volume (0.1 ml) of physiological saline and Freund's complete adjuvant is inoculated into mice subcutaneously in the back or the abdomen or intraperitoneally, 3 to 6 times every 2 to 3 weeks.

Individuals having a high antibody titer are selected from these immunized animals such as mice, and their spleens or lymphatic nodes are recovered therefrom 3 to 5 days after the final immunization. Then, antibody-producing cells contained therein are fused with myeloma cells. The fusing operation may be conducted according to methods known in the art. Fusogens include polyethylene glycol (hereinafter occasionally referred to as PEG) and Sendai virus, and PEG is preferably used. The myeloma cells include NS-1, P3U1 and Sp2/0. In particular, NS-1 and P3U1 are preferably used. For example, the preferable ratio of the number of the spleen cells to that of the myeloma cells is 1:1 to 10:1. It is preferred that PEG having a molecular weight of about 1,000 to 9,000 is added thereto in a concentration of about 10 to 80%, and that the resulting mixture is incubated at about 20 to 37°C, and preferably at 30 to 37°C, for about 3 to 10 minutes.

Various methods can be used for screening of the anti-fibrin antibody-producing hybridoma cells. For example, a microplate is allowed to adsorb fibrinogen, and then thrombin is reacted therewith to convert fibrinogen to a fibrin. Then, the antibody titer of a culture supernatant is determined by an enzyme immunoassay in which the hybridoma culture supernatant is added to the fibrin-fixed microplate in the presence of excess fibrinogen, and an anti-fibrin specific antibody bound to the microplate is detected. Hybridoma cells having positive antibody activity which are selected in HAT (hypoxanthine, aminopterin and thymidine) medium are immediately subjected to cloning, which can be conducted by the limiting dilution method. The antibody titer of the cloned hybridoma culture supernatant is determined by the above-mentioned method and hybridoma cells which stably produce antibodies having a high titer are selected. Thus, the desired monoclonal anti-fibrin specific antibody-producing hybridoma cells can be obtained.

Examples of the anti-fibrin specific antibody-producing hybridoma cells prepared according to the above methods include FIB 1-11 and FIB 2-11 shown in Reference Example 10 described below.

Various methods can also be used for screening of the anti-activated platelet MoAb-producing hybridoma cells. For example, resting platelets or thrombin-activated platelets are bound to a microplate, and fixed with 1% formalin to use as antigens. A hybridoma culture supernatant is added thereto, and the antibody titer of the culture supernatant is determined by an EIA in which the anti-activated platelet antibody bound to the microplate is detected by a second enzyme-labeled antibody. Then, hybridoma cells having large differences between bindings to the resting platelets and to the activated platelets are selected. For example, hybridoma cells having positive antibody activity which are selected in HAT medium are immediately subjected to cloning, which can be conducted by the limiting dilution method. The antibody titer of the cloned hybridoma culture supernatant is determined by the above-mentioned method, and hybridoma cells which stably produce antibodies having a high titer are selected. Thus, the desired monoclonal anti-activated platelet specific antibody-producing hybridoma cells can be obtained.

Examples of the anti-activated platelet MoAb-producing hybridoma cells prepared according to the above-mentioned methods include mouse hybridoma 2T60 shown in Reference Example 14 described below.

Furthermore, the screening of the hybridoma cells which produce the MoAb to the substance having thrombolytic activity (anti-thrombolytic substance MoAb) can be carried out by an EIA using a microplate by which the substance is adsorbed. The cloning is also conducted according to the known methods described above. Thus, the desired anti-thrombolytic substance MoAb-producing hybridoma cells can be obtained.

Examples of the anti-TPA MoAb-producing hybridoma cells prepared by the above methods include mouse hybridoma TPA 1-41, TPA 1-70 and TPA 2-14 shown in Reference Example 11 described below. Examples of the anti-UK MoAb-producing hybridoma cells include mouse hybridoma UK 1-3 and UK 1-87 shown in Reference Example 12 described below, and mouse hybridoma UK 1-6 shown in Reference Example 13.

There are several methods to prepare the polydoma cells which can be used to produce the bispecific hybrid MoAbs of the present invention [for example, H. Shinmoto et al., Proteins, Nucleic Acids, Enzymes 33, 217 (1988)], and any methods may be used. Examples thereof include the following methods:

(1) The above HAT-resistant anti-thrombolytic active substance antibody-producing hybridoma cells are conditioned stepwise in a culture solution containing 5-bromodeoxyuridine (hereinafter occasionally referred to as 5-BrdU), whereby the thymidine kinase-deficient strain is cloned to turn it HAT-sensitive. Similarly, the HAT-resistant ant-fibrin or anti-activated platelet specific antibody-producing hybridoma cells are made 8-azaguanine-resistant (8-azaguanine is hereinafter occasionally referred to as 8-AZG), and the hypoxanthine-guanine-phosphoribosyl transferase-deficient strain is cloned to turn it HAT-sensitive. Then, both are fused with each other according to a standard method. The resulting tetraoma cells are selected in HAT medium, and then the tetraoma cells are cloned which secrete hybrid MoAbs having affinity for both the thrombus and the thrombolytic substance.

(2) The anti-fibrin or anti-activated platelet specific antibody-producing hybridoma cells are labeled with fluorescein isothiocyanate (hereinafter occasionally referred to as FITC), and the anti-thrombolytic substance antibody-producing hybridoma cells are labeled with tetramethylrhodamine isothiocyanate (hereinafter occasionally referred to as TRITC). Then, both are fused with each other by a standard method. The resulting cell suspension is subjected to a fluorescein activated cell sorter (hereinafter occasionally referred to as FACS) to select and clone tetraoma cells having both green fluorescence of FITC and red fluorescence of TRITC. It is also possible to use the markers of both parent strains in reverse, thereby selecting and cloning the tetraoma cells.

For cell fusion in this manipulation, fusogens such as Sendai virus and PEG, or methods such as electrical stimulation are used. PEG is preferably used. One example thereof will hereinafter be described, but the scope of the present invention is not limited thereto. Namely, PEG having a molecular weight of about 1,000 to 9,000 is used in a concentration of about 10 to 80%. The reaction time is about 0.5 to 30 minutes. For example, PEG 6,000 is allowed to contact with cells in a concentration of about 35 to 55% at 37°C for about 4 to 10 minutes to perform the fusion efficiently.

Selection of the polydoma cells can be carried out in the above HAT medium. The polydoma cells are conditioned with drugs such as 8-AZG, 6-thioguanine (hereinafter referred to as 6-TG) and 5-BrdU to obtain the respective drug-resistant strains. Further, various selection media are used by the introduction of new markers into fused cells. Examples of such media include neomycin-added media and hygromycin B-added media [B. Sugden et al., Mol. Cell. Biol., 5, 410 (1985)].

Further, as described above, a method may be used in which the hybridoma cells each labeled with different fluorescent dyes are fused with each other and the hybrid hybridoma cells double labeled are sorted using an FACS [L. Karawajew et al., J. Immunol. Methods, 96, 265 (1987)].

The hybrid antibody-producing polydoma cells can be screened by various methods. For example, the following methods or their modified methods can be suitably used alone or in combination:

(1) A combination of the EIA for screening the above-mentioned anti-fibrin or anti-activated platelet specific antibody-producing hybridoma cells and the EIA for screening the anti-thrombolytic substance antibody-producing hybridoma cells;

(2) An EIA for detecting a bispecific hybrid antibody by adding a culture supernatant to be tested to a fibrin-bound or activated platelet-bound microplate, and then adding an HRP-labeled thrombolytic substance thereto; and

(3) When an anti-thrombolytic substance antibody belonging to a subclass different from that of an anti-fibrin or anti-activated platelet specific antibody is used, an EIA for detecting a bispecific antibody by adding a culture supernatant to be tested to a fibrin-bound or activated platelet-bound microplate, and then adding the HRP-labeled anti-mouse IgG subclass specific antibody thereto.

Polydoma cells having positive antibody activity are immediately subjected to cloning, which can be conducted using a limiting dilution method. The antibody titer of the cloned polydoma culture supernatant is determined by the above-mentioned method, and polydoma cells which stably produce antibodies having a high titer are selected. Thus, the desired monoclonal hybrid antibody-producing polydoma cells can be obtained.

Usually, the above-mentioned polydoma cells of the present invention can be cultivated in liquid media or in peritoneal cavities of animals (for example, in peritoneal cavities of mammals such as mice) by methods known in the art. The antibodies in culture solutions or ascites fluid can be purified using biochemical techniques known in the art in combination. For example, a cell culture solution or ascites fluid is centrifuged to obtain a supernatant. The supernatant is removed and subjected to salt precipitation (usually ammonium sulfate or sodium sulfate is used). The resulting protein precipitate is dissolved in an appropriate solution. After dialysis, the solution is submitted to column chromatography such as ion-exchange column, gel filtration column, protein A column, hydroxyapatite column, ABx column, hydrophobic carrier column or antigen binding column chromatography. Thus, the desired antibody can be separated and purified. By such separation and purification procedures, for example, about 1 to 5 mg of the hybrid MoAb having a purity of 90% or more by protein weight ratio can be obtained from 1 liter of the culture supernatant. Further, aobut 3 to 10 mg of a similar antibody can be obtained from 20 ml of ascites fluid.

Examples of the anti-fibrin-anti-TPA bispecific antibody-producing tetraoma cells prepared by the methods described above include FT 2-1 shown in Reference Example 15, and examples of the anti-fibrin-anti-UK bispecific antibody-producing tetraoma cells include FU 1-74 and FU 2-16 shown in Reference Examples 16 and 17, respectively. Examples of the anti-activated platelet-anti-UK bispecific antibody-producing trioma cells include UP 4-33 shown in Reference Example 19, and examples of the anti-activated platelet-anti-UK bispecific antibody-producing tetraoma cells include UP 3-175 shown in Reference Example 18.

There are several methods to prepare the bispecific antibodies of the present invention having the variable regions and lacking $CH_2$ and $CH_3$.

(1) A bispecific antibody produced by the above-mentioned polydoma is subjected to enzymolysis to prepare an antibody having a variable region related to binding to two kinds of antigens and lacking $CH_2$ and $CH_3$. Enzymes used in this case include pepsin [A. Nisonoff et al., Science, 132, 1770 (1960)], papain [R. R. Porter et al., Biochem. J., 73, 119 (1959)], ficin [M. Mariani et al., Mole. Immunol., 28, 69 (1991)], bromelin [D. E. Milenic et al., J. Immunol. Methods, 120, 71 (1989)], elastase and chymotrypsin. Pepsin and chymotrypsin are preferably used.

(2) Each of a MoAb specific for human fibrin or activated platelets and a MoAb specific for a thrombolytic substance is subjected to the enzymolysis shown in (1) to prepare an antibody protein fraction having monovalent antigen binding activity such as Fab or Fab'. Next, an antibody protein fraction having different specificity is chemically rebound thereto to prepare a bispecific antibody containing a desired variable region and lacking $CH_2$ and $CH_3$. In this case, the method of treating two kinds of MoAbs with pepsin or ficin, and then reducing to Fab's, followed by chemical reassociation thereof to prepare Fab' heterodimers is preferably used. In this reassociation reaction, substituent groups contained in antibody molecules, such as amino, carboxyl, hydroxyl and sulfhydryl groups, can be utilized. For example, the following methods are used:

(a) A reactive amino group contained in one antibody is condensed by dehydration with a reactive carboxyl group contained in another antibody in an aqueous solvent using a water-soluble carbodiimide reagent [for example, 1-ethyl-3-(3-dimethylaminopropyl)-carbodiimide or 1-cyclohexyl-3-(2-morpholinoethyl)-carbodiimide or p-toluene sulfonate].

(b) A reactive amino group contained in one antibody is reacted with an active ester of N-hydroxysuccinamide [for example, an ester of p-maleimidomethylcyclohexane-1-carboxyl-N-hydroxysuccinimide or an ester of N-($\epsilon$-maleimidocaproyloxy)succinimide ester] to maleimidate the amino group, and then the resulting maleimidated amino group is bound by a thioether bond to (i) another antibody reduced with dithiothreitol (DTT) or (ii) another antibody into which a sulfhydryl group is introduced using SPDP.

(c) Both reactive amino acid groups of two kinds of antibodies are bound to each other using a dialdehyde reagent such as succindialdehyde or glutaraldehyde.

(d) Two kinds of antibodies are reduced with DTT or sulfhydryl groups are introduced into the antibodies using SPDP, followed by reoxidation to prepare a heterodimer. Desired heterodimeric antibodies can be efficiently prepared with impairing the activity of two kinds of antibodies as little as possible by various combinations of these methods [M. J. Glennie et al., J. Immunol., 139, 2367 (1987); and T. Kitagawa, Organic Synthetic Chemistry, 42, 283 (1984)].

(3) A variable region-containing single-chain MoAb specific for human fibrin or activated platelets and a variable region-containing single-chain MoAb specific for thrombolytic substance are each prepared by recombinant technology known in the art [J. S. Huston et al., Proc. Natl. Acad. Sci. USA, 85, 5879 (1988); and R. E. Bird et al., Science, 242, 423 (1988)], and then these two kinds of single-chain antibodies having different specificity are associated with each other by the chemical binding method shown in (2) to prepare a desired bispecific antibody.

(4) An Fab or Fab' fraction of a variable region-containing single-chain MoAb specific for human fibrin or activated platelets and an Fab or Fab' fraction of a variable region-containing single-chain MoAb specific for thrombolytic substance are each prepared by recombinant technology known in the art [R. L. Mullinax et al., Proc. Natl. Acad. Sci. USA, 87, 8095 (1990)], and then these two kinds of Fab or Fab' fractions having different specificity are bound to each other by the chemical method shown in (2) to prepare a desired bispecific antibody.

When the antibodies of the present invention are derived from antibodies of animals other than humans, such as mice, regions other than hypervariable regions of the proteins, for example, constant regions or frame work regions, can be converted to human antibody-derived regions using gene manipulation technology [Z. Steplewski et al., Proc. Natl. Acad. Sci. USA, 85, 4852 (1988)], whereby a mouse-human chimera type or humanized proteins can also be prepared. When given to humans, such humanized antibodies are favorably used because of their weak antigenicity.

In thrombolytic therapeutics using selective thrombolytic agent prepared from the bispecific hybrid MoAbs lacking $CH_2$ and $CH_3$ of the present invention, or the substances having thrombolytic activity and the bispecific hybrid MoAbs, several methods are used. Examples thereof include the following methods:

(1) A hybrid MoAb of the present invention is preliminarily given to patients with thrombotic diseases, and a thrombolytic substance such as TPA, UK or ProUK is given, after a sufficient time to ligate the MoAb to thrombi formed in the bodies of the patients has elapsed.

(2) The hybrid MoAb and the thrombolytic substance are simultaneously given at a molar ratio of 5:1 to 1:1 to patients with thrombotic diseases.

(3) The hybrid MoAb is preliminarily reacted with the thrombolytic substance, preferably at a molar ratio of 1:1, and the unreacted thrombolytic substance is removed. Then, the resulting selective thrombolytic protein complex is given to patients with thrombotic diseases.

The thrombolytic agents of the present invention, the hybrid proteins or the thrombolytic substances, are formed into preparations such as injections, as themselves alone or as mixtures of them with pharmaceutically acceptable carriers, excipients, diluents and the like, after filtration and sterilization procedures with membrane filters if necessary, and are given to mammals such as mice, rats, cats, dogs, pigs, cattle, monkeys and humans. They can be used for treatment of thrombotic obstructive diseases such as cardiac infarction, peripheral arterial or venous obstruction, retinal arterial or venous obstruction, cerebral infarction and pulmonary embolism.

The dosage of the thrombolytic agents of the present invention varies depending on the subject disease, symptom, route of administration and the like. For example, when the thrombolytic agent is intravenously given to adult patients with cardiac infraction, the bispecific MoAb lacking $CH_2$ and $CH_3$ is used in an amount of about 0.02 to 1 mg/kg daily, preferably about 0.04 to 0.4 mg/kg daily, and the thrombolytic substance is used in an amount of about 0.01 to 0.5 mg/kg daily, preferably about 0.02 to 0.2 mg/kg daily, for TPA, in an amount of about 0.01 to 0.5 mg/kg, preferably about 0.02 to 0.2 mg/kg, for UK, and in an amount of about 0.01 to 1 mg/kg, preferably about 0.02 to 0.5 mg/kg, for ProUK.

According to the methods described above, the thrombi can be lysed and removed selectively and efficiently by using the hybrid MoAb of the present invention which specifically binds to a target thrombotic site and does substantially bind to fibrinogen contained in blood or resting platelets, and the thrombolytic substance.

The bispecific antibodies of the present invention having the variable regions and lacking heavy chain constant region domains 2 and 3 do not substantially react with fibrinogen or the resting platelets, and specifically bind to the fibrins forming thrombi or the activated platelets. Similarly, the antibodies can specifically bind also to the thrombolytic substances without impairing their fibrinolysis activity. It is therefore possible to prepare the 1:1 immune complexes of the antibodies and the thrombolytic substances. By using both in combination, the thrombolytic agents are provided which are extremely reduced in their side effects associated with acceleration of fibrinogen degradation and $\alpha_2$-antiplasmin consumption, and which lyse or remove the thrombi selectively and efficiently.

The present invention will hereinafter be described in detail with the following Reference Examples, Examples and Test Examples. It is understood of course that these are not intended to limit the scope of the invention.

The animal cells used in Reference Examples and Examples are deposited in the deposition institutes as shown in the following table:

| Animal cell | IFO (IFO No.) | FRI (FERM No.) |
|---|---|---|
| Mouse hybridoma FIB 1-11 | 50174 (Sept.21,1988) | BP-2081 (Oct.4,1988) |
| Mouse hybridoma FIB 2-11 | 50175 (Sept.21,1988) | BP-2082 (Oct.4,1988) |
| Mouse hybridoma TPA 1-41 | 50178 (Sept.21,1988) | BP-2085 (Oct.4,1988) |
| Mouse hybridoma TAP 1-70 | 50179 (Sept.21,1988) | BP-2086 (Oct.4,1988) |
| Mouse hybridoma TAP 2-14 | 50194 (July 14,1988) | BP-2519 (July 18,1990) |
| Mouse hybridoma UK 1-3 | 50176 (Sept.21,1988) | BP-2083 (Oct.4,1988) |
| Mouse hybridoma UK 1-87 | 50177 (Sept.21,1988) | BP-2084 (Oct.4,1988) |
| Mouse hybridoma UK 1-6 | 50208 (Aug.9,1988) | BP-2548 (Aug.11,1989) |
| Mouse hybrid hybridoma (Tetraoma) FT 2-14 | 50180 (Nov.8,1988) | BP-2158 (Nov.25,1988) |
| Mouse hybridoma FU 1-74 | 50185 (May 13,1988) | BP-2334 (May 14,1989) |
| Mouse hybridoma FU 2-16 | 50207 (Aug.9,1988) | BP-2547 (Aug.11,1989) |
| Mouse hybridoma 2T60 | 50211 (Sept.27,1989) | BP-2623 (Oct.4,1989) |
| Mouse hybrid hybridoma UP 3-175 | 50224 (Feb.21,1990) | BP-2845 (May 30,1990) |
| Mouse hybrid hybridoma UP 4-33 | 50251 (July 3,1989) | BP-3018 (July 13,1990) |

IFO: The Institute for Fermentation (Osaka) (17-85, Jusohonmachi 2-chome, Yodogawa-ku, Osaka, Japan) FRI: The Fermentation Research Institute, the Agency of Industrial Science and Technology, the Ministry of International Trade and Industry (1-3, Higashi 1-chome, Tsukuba-shi, Ibaragi-ken, Japan)

In this specification, amino acids and peptides are indicated by abbreviations adopted by the IUPAC-IUB Commission on Biochemical Nomenclature (CBN). For example, the following abbreviations are used. When the amino acids are capable of existing as optical isomers, it is understood that the L-forms are represented unless otherwise specified.

Gln:    Glutamine residue
Asp:    Aspartic acid residue
Pro:    Proline residue
Tyr:    Tyrosine residue
Val:    Valine residue
Lys:    Lysine residue
Glu:    Glutamic acid residue
Ala:    Alanine residue
Asn:    Asparagine residue
Leu:    Leucine residue
Phe:    Phenylalanine residue
Gly:    Glycine residue
His:    Histidine residue
Ser:    Serine residue
Thr:    Threonine residue
Ile:    Isoleucine residue
Trp:    Tryptophan residue
Arg:    Arginine residue
Met:    Methionine residue
Cys:    Cysteine residue

Reference Example 1

EIA for Measuring Anti-Fibrin Antibody

A 1 mg/l human fibrin monomer solution in phosphate buffered saline (PBS, pH: 7.3) supplemented with 3.3 M urea and 0.01% EDTA was added to a 96-well microplate in an amount of 50 µl/well and allowed to stand overnight at 4°C. Then, 150 µl of PBS supplemented with 2% casein and 0.01% thimerosal was added thereto to prepare a sensitized plate. Thereafter, a 10 mg/ml human fibrinogen solution in PBS supplemented with 100 units/ml heparin and 3 mM phenylmethylsulfonyl fluoride was mixed with an equal volume of a test hybridoma culture supernatant. After reaction at room temperature for 30 minutes, 100 $\mu$l/well of the reaction solution was added to the above-mentioned fibrin-sensitized plate, followed by reaction at room temperature for 2 hours. The plate was thoroughly washed with PBS supplemented with 0.05% Tween 20 (hereinafter occasionally referred to as PBS-Tw), and then horseradish peroxidase (HRP)-labeled rabbit anti-mouse IgG antibody was added thereto, further followed by reaction at room temperature for 2 hours.

After washing, 0.1 M citrate buffer containing o-phenylenediamine and $H_2O_2$ was added to each well as a substrate, and enzyme reaction was conducted at room temperature. The reaction was terminated with 1 N sulfuric acid, and then the amount of color-developed dye was determined at 492 nm with Multiscan (Flow Laboratory).

Reference Example 2

EIA for Measuring Anti-TPA Antibody

A 5 $\mu$g/ml commercial single-chain TPA solution (sold by Chuo Kagaku Kogyo) was added to a 96-well microplate in an amount of 100 $\mu$l/well and allowed to stand overnight at 4°C. Then, 150 $\mu$l/well of PBS containing 2% casein and 0.01% thimerosal was added thereto to prepare a sensitized plate. The above solution was removed from the microplate and the microplate was washed with PBS-Tw. Then, 100 $\mu$l/well of a test hybridoma culture supernatant was added thereto and reacted at room temperature for 2 hours. Thereafter, enzyme reaction was conducted according to the method described in Reference Example 1 and the antibody titer was assayed.

Reference Example 3

EIA for Measuring Anti-UK Antibody

A UK-sensitized plate was prepared in the same manner as with Reference Example 2 with the exception that UK (manufactured by Nippon Seiyaku) was substituted for TPA, and the anti-UK antibody titer was similarly assayed.

Reference Example 4

EIA for Measuring Anti-Low Molecular Weight UK Antibody

A low molecular weight UK-sensitized plate was prepared in the same manner as with Reference Example 2 with the exception that low molecular weight UK (two-chain low molecular weight UK, sold by JCR) and the anti-low molecular weight UK antibody titer was similarly assayed.

Reference Example 5

EIA for Measuring Anti-Platelet Antibody

(1) Preparation of Fixed Platelets

Platelet-rich plasma was obtained by centrifugation from fresh human blood collected by using sodium citrate, and washed with Tyrode-Hepes buffer (pH 6.5) containing an ADP-hydrolyzing enzyme. The washed platelets were seeded in a microplate in an amount of $2 \times 10^7$ platelets/well, and activated with thrombin (0.2 units/ml), followed by centrifugation. Then, after fixing with 2% formalin, the plate was blocked with PBS containing 5% bovine serum albumin (hereinafter occasionally referred to as BSA) to prepare an activated platelet plate. A resting platelet plate was prepared in the same manner as above with the exception that the thrombin activating procedure was omitted.

(2) EIA Procedure

To the platelet plate was added 100 $\mu$l/well of a hybridoma culture supernatant, and reacted at room temperature for 3 hours, followed by washing with PBS-Tw. Thereafter, the anti-platelet antibody titer was assayed in a manner similar to that described in Reference Example 1.

Reference Example 6

EIA for Measuring Anti-Fibrin-Anti-TPA Hybrid Antibody

A hybrid antibody-containing test solution was added to the TPA-sensitized plate prepared in Reference Example 2 and reacted at room temperature for 2 hours. After washing with PBS-Tw, a human fibrin $\beta$-chain N-terminal peptide (1-11)-BSA complex described in Reference Example 10-(1) was labeled with biotin and added thereto, followed by further reaction at room temperature for 2 hours. Then, an avidin-HRP complex was added thereto, followed by reaction at room temperature for 1 hour. Thereafter, the activity of HRP bound to the solid phase was assayed by the method described in Reference Example 1.

Reference Example 7

EIA for Measuring Anti-Fibrin-Anti-UK Hybrid Antibody

A hybrid antibody-containing test solution was added to the UK-sensitized plate prepared in Reference Example 3, and thereafter the bispecific antibody titer was assayed in a manner similar to that described in Reference Example 6.

Reference Example 8

EIA for Measuring Anti-Activated Platelet-Anti-UK Hybrid Antibody

A test hybridoma culture supernatant was added to the activated platelet-sensitized plate prepared in Reference Example 5 and reacted at room temperature for 2 hours. After washing with PBS-Tw, biotin-labeled UK was added thereto and further reacted at room temperature for 2 hours. Then, an avidin-HRP complex was added thereto, followed by reaction at room temperature for 1 hour. Thereafter, the activity of HRP bound to the solid phase was assayed by the method described in Reference Example 1.

Reference Example 9

Neutralization Test of Fibrinolysis Reaction

A diluted solution of a test hybridoma culture supernatant was added to a TPA solution (final concentration: 20 ng/ml) or a UK solution (final concentration: 25 ng/ml), and reacted at 37°C for 1 hour. Then, the reaction mixture was poured into each well of a fibrin agarose plate in an amount of 5 $\mu$l. After standing at 37°C for 2 to 6 hours, the diameter of the fibrinolysis plaque was measured to determine the neutralization activity of a MoAb contained in the hybridoma culture supernatant to the enzyme activity of TPA or UK.

Reference Example 10

Preparation of Mouse Anti-Human Fibrin Monoclonal Antibody-Producing Hybridomas

(1) Preparation of Immunogen

To a 12 mg/2 ml solution of BSA preliminarily maleimidated with GMBS (maleimido groups were introduced into BSA in an amount of 13 mol/mol of BSA), 3.3 mg of human fibrin $\beta$-chain N-terminal peptide (1-11)-Cys prepared by a known solid-phase synthesizing method using a peptide synthesizer (Model 430A type, Applied System) was added, and reacted at 30°C for 1 hour to obtain a human fibrin $\beta$-chain N-terminal peptide (1-11)-BSA complex. Then, the complex was dialyzed 3 times against physiological saline (3 liters x 3 times), followed by freeze-storage to use it as an immunogen

(2) Immunization

An equal volume of Freund's complete adjuvant was added to a 1 mg/ml solution of the peptide-BSA complex in physiological saline. The mice (females, n = 10, 1 mg/0.2 ml/mouse) started to be immunized with the resulting mixture subcutaneously in their backs and abdomens. Additional immunization was carried out 3 times at intervals of 2 to 3 weeks, using a mixture of the immunogen and an equal volume of Freund's incomplete adjuvant.

(3) Cell Fusion

The spleen was taken out of the mouse 3 days after the final immunization, and a spleen cell suspension containing about $10^8$ cells was prepared by a standard method. Then, $2 \times 10^7$ mouse myeloma cells (P3U1) were added thereto, and cell fusion was conducted using PEG 6000 according to the method of Köller and Milstein [Nature, 256, 495 (1975)].

After completion of the fusion, the cell mixture was suspended in HAT medium and cultivated for 10 days. Then, immediately after selection of parent cells was completed, HT medium from which aminopterin was eliminated was substituted for HAT medium, and the cultivation was continued.

(4) Selection and Cloning of Hybridomas

The antibody titer of the hybridoma culture supernatants was determined by the EIA of Reference Example 1 using the microplate in which the human fibrin monomer was allowed to be adsorbed by the solid phase. After 10 to 20 days from the fusion, hybridoma cells and an antibody which specifically bind to human fibrin were observed. The hybridoma cells having particularly high binding activity were cloned by the limiting dilution method.

Similarly, the cloned hybridoma culture supernatants were screened by the EIA, and hybridoma cells having high binding activity to human fibrin were selected. As a result, hybridomas FIB 1-11 and FIB 2-11 producing a MoAb which could specifically binds to the fibrin in the presence of high concentrated human fibrinogen were obtained. Their immunoglobulin class and subclass were examined by the Ouchterlony method. They were both found to belong to $IgG_1$.

Reference Example 11

Preparation of Mouse Anti-TPA Monoclonal Antibody-Producing Hybridomas

(1) Immunization

To a 200 $\mu$g/l solution of TPA in physiological saline, an equal volume of Freund's complete adjuvant was added and fully emulsified. The emulsion was then given to the BALB/c mice (females, 20 $\mu$g/0.2 ml/mouse) intraperitoneally and subcutaneously in their backs, and additional immunization was carried out at intervals of 2 to 3 weeks. After the additional immunization was conducted 3 times, a TPA antigen solution (50 $\mu$g/0.1 ml of physiological saline/mouse) was intravenously given to the individual which showed the highest serum antibody titer after 10 days.

(2) Cell Fusion

Cell fusion was conducted according to the method described in Reference Example 10-(3).

(3) Selection and Cloning of Hybridomas

Hybridoma cells were screened by the EIA described in Reference Example 2 using the TPA-bound microplate, and anti-TPA MoAb-producing hybridoma cells were obtained in the same manner as with Reference Example 10-(4). Of these, mouse hybridoma TPA 1-41 was obtained as a hybridoma cell which produces an anti-TPA MoAb having binding specificity to TPA without impairing its fibrinolysis activity, mouse hybridoma TPA 1-70 was obtained as anti-TPA MoAb-producing hybridoma cells which produce MoAb having TPA neutralization activity in the neutralization test of fibrinolysis reaction described in Reference Example 9, and mouse hybridoma TPA 2-14 was obtained as hybridoma cells which produce TPA non-neutralization anti-TPA MoAb having competitive binding activity with plasminogen activator inhibitor (PAI) to TPA. The immunoglobulin class and subclass of antibodies TPA 1-14, TPA 1-70 and TPA 2-14 each produced from the resulting hybridoma cells were found to be $IgG_{2b}$, $IgG_1$ and $IgG_1$, respectively, by the Ouchterlony method.

Reference Example 12

Preparation of Mouse Anti-UK Monoclonal Antibody-Producing Hybridomas

(1) Immunization

The mice were immunized in the same manner as with Reference Example 11-(1) with the exception that UK is substituted for TPA.

(2) Cell Fusion

Cell fusion was carried out according to the method described in Reference Example 10-(3).

(3) Selection and Cloning of Hybridomas

Hybridoma cells were screened by the EIA described in Reference Example 3 using the UK-bound microplate, and anti-UK MoAb-producing hybridoma cells were obtained in the same manner as with Reference Example 10-(4). Of these, mouse hybridomas UK 1-3 and UK 1-87 were obtained as anti-UK MoAb-producing hybridoma cells which produces a MoAb having binding specificity to UK without impairing its fibrinolysis activity. The immunoglobulin class and subclass of antibodies UK 1-3 and Uk 1-87 each produced from the resulting hybridoma cells were found to be $IgG_1$ and $IgG_{2b}$, respectively, by the Ouchterlony method.

Reference Example 13

Preparation of Mouse Anti-Low Molecular Weight UK Monoclonal Antibody-Producing Hybridoma

(1) Immunization

The mice were immunized in the same manner as with Reference Example 11-(1) with the exception that low molecular weight UK is substituted for TPA

(2) Cell Fusion

Cell fusion was carried out according to the method described in Reference Example 10-(3).

(3) Selection and Cloning of Hybridoma

Hybridoma cells were screened by the EIA described in Reference Example 4 using the low molecular weight UK-bound microplate, and low molecular weight UK MoAb-producing hybridoma cells were obtained in the same manner as with Reference Example 10-(4). Of these, mouse hybridoma UK 1-6 was obtained as a hybridoma which produces an anti-low molecular weight UK MoAb having binding specificity to UK without impairing its fibrinolysis activity in the method described in Reference Example 9. The immunoglobulin class and subclass of antibody UK 1-6 produced from the resulting hybridoma were found to be $IgG_1$ ($x$ chain) by the Ouchterlony method.

Reference Example 14

Preparation of Mouse Anti-Activated Platelet Antibody-Producing Hybridoma

(1) Immunization

Washed platelets were obtained by centrifugation from fresh human blood collected using citric acid.
To about $10^9$ platelets, 0.1 unit/ml of thrombin was added and incubated at 37°C for 5 minutes, followed by intraperitoneal injection to the BALB/c mice. Immunization was carried out 6 to 8 times at intervals of 2 weeks.

(2) Cell Fusion

Cell fusion was carried out in the same manner as with Reference Example 10-(3) with the exception

12

that NS-1 is substituted for mouse myeloma cell P3U1.

The immunoglobulin class and subclass of antibody 2T60 was found to be IgG$_1$ (x chain) by the Ouchterlony method.

## (3) Selection and Cloning of Hybridoma

Hybridoma cells were screened by the EIA of Reference Example 5 using the platelet-bound microplate, and anti-activated platelet MoAb-producing hybridoma was obtained in the same manner as with Reference Examples 10 - (4). As a result, hybridoma 2T60 which produces a MoAb specifically binding to activated human platelets and activated rabbit platelets was obtained.

## Reference Example 15

Preparation of Hybrid Monoclonal Antibody Having Anti-TPA-Anti-Human Fibrin Bispecificity

### (1) Cell Fusion

Anti-human fibrin antibody-producing hybridoma FIB 1-11 obtained in Reference Example 10 and anti-TPA antibody-producing hybridoma TPA 1-41 obtained in Reference Example 11 were each incubated in Iskove-Ham F-12 mixed medium supplemented with 0.5 $\mu$g/ml FITC and 1.5 $\mu$g/ml TRITC, at 37°C for 30 minutes to fluorescent stain them. Then, an LSM solution (sold by Wako Pure Chemical Industries) was added thereto to remove dead cells, and thereafter both hybridoma cells were mixed with each other in a ratio of 1:1. Using PEG 6000, cell fusion was conducted by the method described in Reference Example 10-(3).

After incubation at 37°C for 2 hours, 25,000 cells were separately taken from cells stained double with fluorescein and rhodamine by subjecting the cells to an FACS. Then,the above-mentioned double-stained cells were seeded in a ratio of 10 cells/well in a 96-well microplate in which 5X10$^5$ cells/well of mouse thymocytes had been seeded, and cultivated.

### (2) Selection and Cloning of Hybrid Hybridoma

Culture supernatants of wells in which cell proliferation was observed 1 to 2 weeks after fusion were subjected to the EIAs described in Reference Examples 1, 2 and 6, respectively, to determine antibody activity.

For wells which exhibited high hybrid antibody activity in all the EIAs, cloning was performed by the limiting dilution method. As a result, desired bispecific antibody-producing mouse hybrid hybridoma (tetraoma) FT 2-14 was obtained.

## Reference Example 16

Preparation of Hybrid Monoclonal Antibody Having Anti-UK-Anti-Human Fibrin Bispecificity (1)

### (1) Cell Fusion

Anti-human fibrin antibody-producing hybridoma FIB 1-11 obtained in Reference Example 10 and anti-UK antibody-producing hybridoma UK 1-3 obtained in Reference Example 12 were each fluorescent stained with FITC and TRITC according to the method described in Reference Example 15-(1), followed by cell fusion using PEG 6000. Then, the fused cells were subjected to an FACS, and the double-stained cells were selected and cultivated.

### (2) Selection and Cloning of Hybrid Hybridoma

Culture supernatants of wells in which cell proliferation was observed 1 to 2 weeks after fusion were subjected to the EIAs described in Reference Examples 1, 3 and 7, respectively, to determine antibody activity.

For wells which exhibited the highest hybrid antibody activity, cloning was performed by the limiting dilution method. As a result, desired bispecific antibody-producing mouse hybrid hybridoma FU 1-74 was obtained.

Reference Example 17

Preparation of Hybrid Monoclonal Antibody Having Anti-UK-Anti-Human Fibrin Bispecificity (2)

(1) Cell Fusion

Anti-human fibrin antibody-producing hybridoma FIB 1-11 obtained in Reference Example 10 and anti-low molecular weight UK antibody-producing hybridoma UK 1-6 obtained in Reference Example 13 were each fluorescent stained with FITC and TRITC according to the method described in Reference Example 15-(1), followed by cell fusion using PEG 6000. Then, the fused cells were subjected to an FACS, and the double-stained cells were selected and cultivated.

(2) Selection and Cloning of Hybrid Hybridoma

Culture supernatants of wells in which cell proliferation was observed 1 to 2 weeks after fusion were subjected to the EIAs described in Reference Examples 1, 4 and 7, respectively, to determine antibody activity.

For wells which exhibited the highest hybrid antibody activity, cloning was performed by the limiting dilution method. As a result, desired bispecific antibody-producing mouse hybrid hybridoma FU 2-16 was obtained.

Reference Example 18

Preparation of Hybrid Monoclonal Antibody Having Anti-UK-Anti-Activated Platelet Bispecificity (1)

(1) Cell Fusion

Anti-activated platelet MoAb-producing hybridoma 2T60 obtained in Reference Example 14 and anti-low molecular weight UK MoAb-producing hybridoma UK 1-6 obtained in Reference Example 13 were each incubated in Iskove-Ham F-12 mixed medium supplemented with 0.5 $\mu$g/ml FITC and 1.5 $\mu$g/ml TRITC, at 37°C for 30 minutes to fluorescent stain them. Then, an LSM solution (sold by Wako Pure Chemical Industries) was added thereto to remove dead cells, and thereafter both hybridoma cells were mixed with each other in a ratio of 1:1. Using PEG 6000, cell fusion was conducted by the method described in Reference Example 15-(1).

After incubation at 37°C for 2 hours, 25,000 cells were separately taken from cells stained double with fluorescein and rhodamine by subjecting the cells to an FACS. Then, the above-mentioned double-stained cells were seeded in a ratio of 10 cells/well in a 96-well microplate in which $5X10^5$ cells/well of mouse thymocytes had been seeded, and cultivated.

(2) Selection and Cloning of Tetraoma

For wells which were positive in the EIA described in Reference Example 8, cloning was performed by the limiting dilution method. As a result, mouse hybrid hybridoma UP 3-175 was obtained which showed high hybrid antibody activity.

Reference Example 19

Preparation of Hybrid Monoclonal Antibody Having Anti-UK-Anti-Activated Platelet Bispecificity (2)

(1) Selection of HAT-Sensitive Strain

Anti-activated platelet specific MoAb-producing hybridoma 2T60 obtained in Reference Example 14 was cultivated in 5 $\mu$M 8-AZG-added medium and subcultured for several weeks, increasing the 8-AZG concentration to 100 $\mu$M stepwise in turn. The strain having the highest anti-activated platelet MoAb producing activity was selected from the resulting 8-AZG-resistant, HAT-sensitive strains, and subjected to cell fusion.

(2) Cell Fusion

The anti-UK MoAb-producing spleen cells obtained in Reference Example 12-(1) were mixed with HAT-sensitive anti-activated platelet MoAb-producing hybridoma 2T60 obtained in (1) described above in a ratio of 1:5, and cell fusion was conducted by the method described in Reference Example 10-(3) using PEG 6000.

(3) Selection and Cloning of Trioma

After completion of the fusion, the cell mixture was suspended in HAT medium, and selectively cultivated in HAT medium according to the method described in Reference Example 10-(3) to prepare desired trioma cells. A culture supernatant of these trioma cells were subjected to the EIA described in Reference Example 3 to select wells exhibiting positive anti-UK antibody activity. Further, the anti-activated platelet-anti-UK hybrid antibody activity was measured by the EIA described in Reference Example 8. For wells showing high bispecific antibody activity, cloning was performed by the limiting dilution method. As a result, mouse hybrid hybridoma UP 4-33 was obtained.

Example 1

Preparation of $F(ab')_2$ Fraction of Anti-UK-Anti-Human Fibrin Bispecific Hybrid Antibody

(1) Purification of Hybrid Antibody

Onto 6 BALB/c mice preliminarily given 0.5 ml of mineral oil intraperitoneally, $5 \times 10^6$ cells/mouse of mouse hybrid hybridoma FU 1-74 were inoculated intraperitoneally. After about 10 to 20 days, a pool of the ascites fluid was observed. The ascites fluid was collected, and subjected to salt precipitation using 45-50% saturated ammonium sulfate to obtain an IgG fraction.

Then, the fraction was subjected to a fibrin-bound Cellulofine column equilibrated with 20 mM PBS, and was eluted with 0.2 M glycine-hydrochloric acid buffer (pH 2.9) to obtain a protein fraction. The resulting protein fraction was neutralized with 1 N NaOH, dialyzed against 10 mM potassium phosphate buffer (pH 6.8), and then applied to high performance liquid chromatography using a hydroxyapatite column equilibrated with the same buffer. Linear gradient elution was accomplished by use of potassium phosphate buffer (pH 6.8) from 10 mM to 210 mM to purify bispecific hybrid antibody FU 1-74.

From about 200 ml of the ascites fluid, 166 mg of purified antibody FU 1-74 was obtained.

(2) Purification of $F(ab')_2$

Purified antibody FU 1-74 thus obtained was dissolved in 20 mM acetate buffer (pH 3.5), and subjected to a pepsin-coupled column (5 mg pepsin/2.5 ml Cellulofine gel). Elution was slowly carried out at 37°C at a rate of 3 ml/hour to obtain a pepsin digest. The pepsin digest was adjusted to pH 7.5 with 1 N NaOH, and then subjected to a protein A column to obtain protein fractions eluted with PBS (pH 7.5). The fractions were further subjected to a fibrin-bound column, and the column was eluted and washed, followed by elution of an $F(ab')_2$ fraction having fibrin binding activity with glycine-hydrochloric acid buffer having a pH of 3.0. From about 150 mg of FU 1-74 (whole IgG molecules), 20 mg of the FU 1-74 $F(ab')_2$ fraction was obtained.

(3) Bispecific Antibody Activity

The $F(ab')_2$ fraction obtained in (2), as well as the whole IgG molecules, was subjected to the EIA described in Reference Example 7 to assay bispecific antibody activity.

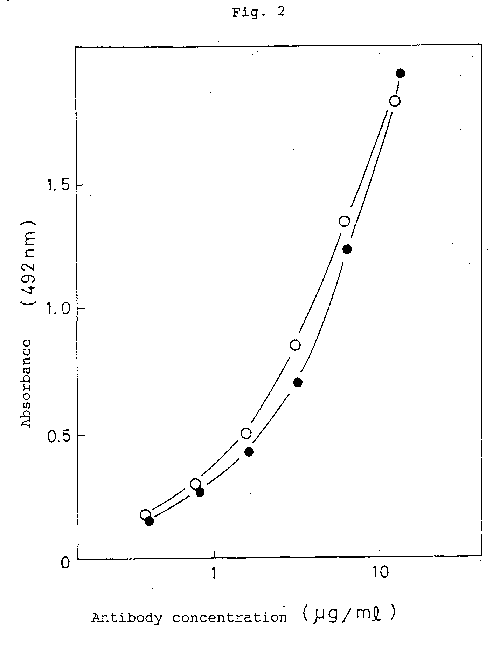

The results are shown in Fig. 2. The $F(ab')_2$ fraction (O) had bispecific antibody activity similar to that of the whole IgG molecules (●).

Example 2

Preparation of Thrombolytic Protein Complex Solution

The $F(ab')_2$ fraction of anti-UK-human fibrin bispecific MoAb FU 1-74 obtained in Example 1-(2) was added to a defined amount of ProUK (final concentration: 250 ng/ml) so as to give a fraction-to-ProUK molar ratio of 0.25:1, 0.5:1, 0.75:1, 1.0:1, 1.5:1 or 2.0:1, and the mixture was subjected to immune reaction at room temperature for 20 minutes to prepare a thrombolytic protein complex solution.

The whole IgG molecules of bispecific MoAb FU 1-74 obtained in Example 1-(1) was similarly treated to prepare a thrombolytic protein complex having an antibody-to-ProUK molar ratio of 0.25:1, 0.5:1, 1.0:1, 1.5:1 or 2.0:1, which was subjected to Test Examples described below.

Test Example 1

Enhancement of ProUK Fibrinolysis Activity (1)

A plasma clot lysis assay was carried out according to the method known in the art [D. Collen et al., Thromb. Haemostasis, 45, 225 (1981)]. Namely, the human plasma was added to a thrombolytic protein complex solution of the whole IgG molecules of bispecific MoAb FU 1-74 and that of the F(ab')$_2$ fraction prepared in Example 2, and then human thrombin was added thereto to a final concentration of 1.0 unit/ml to coagulate the plasma.

Using an euglobulin lysis analyzer ("ELT-6", Mebanix Co.), the turbidity of the plasma was observed with time to measure the time required for dissolution.

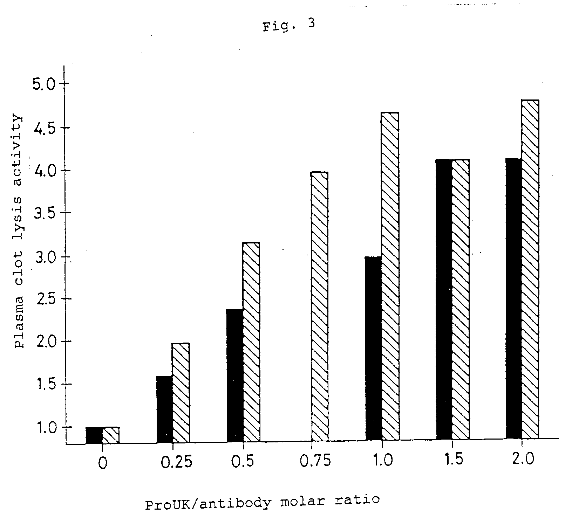

Results thereof are shown in Fig. 3. The plasma lysis activity was enhanced by addition of the F(ab')$_2$ fraction (shaded) to ProUK, and approximately reached a plateau at a molar ratio of 1:1. The effect of enhancing the lysis activity of ProUK exhibited activity similar to or stronger than that of the whole IgG molecules of original FU 1-74 (closed).

Test Example 2

Enhancement of ProUK Fibrinolysis Activity (2)

(1) Preparation of $^{125}$I-Labeled Plasma Clot

Commercial $^{125}$I-labeled human fibrinogen (10 $\mu$g/10 $\mu$l, sold by Muromachi Kagaku Kogyo) was added to 600 $\mu$l of the human plasma, and then bovine thrombin (1 unit/100 $\mu$l) was added thereto, followed by rapid stirring. The resulting solution was sucked into a catheter treated with 10% Tween 80, and allowed to stand at room temperature for 1 minute, followed by incubation at 37°C for 30 minutes. The resulting plasma clot was squeezed onto a plate supplied with physiological saline, and cut at 1 cm intervals with a knife. The radioactivity of each section was measured by a $\gamma$-counter.

(2) Model Test of Hamster Pulmonary Embolus

After the hamster (body weight: 80 to 100 g) was intraperitoneally given pentobarbital (6 mg/0.3 ml), a catheter for blood collection was inserted into the femoral vein. Then, the $^{125}$I-labeled plasma clot prepared in (1) was sucked into a catheter and injected into the carotid artery, followed by insertion of a catheter for giving a sample. NaI (0.2 mg/0.1 ml) and heparin (100 units/0.1 ml) were given through the carotid artery, and then 350 $\mu$l of ProUK alone or a thrombolytic protein complex prepared by adding 2-fold mol of bispecific MoAb FU 1-74 described in Reference Example 16 or the F(ab')$_2$ fraction obtained in Example 1-(2) to ProUK and treating the mixture in accordance with the method described in Example 2 was further given. After standing at room temperature for 90 minutes, the blood (1 ml) was collected, and the breast was opened to remove the right lung, the left lung and the heart. The radioactivity of each organ was measured by a $\gamma$-counter. The lysis rate of the plasma clot was determined from the ratio of the residual amount of radioactivity for the three organs to the total amount of given radioactivity.

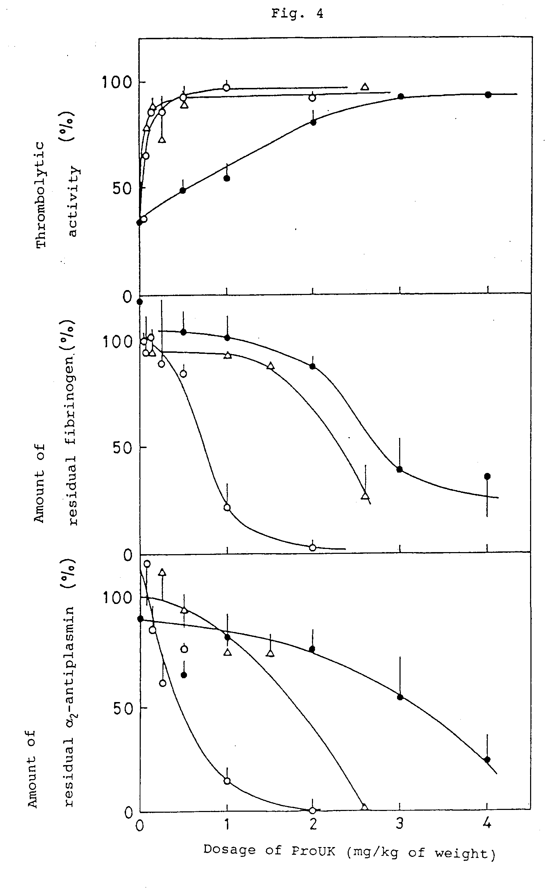

Results thereof are shown in Table 1 and Fig. 4.

Table 1

| Given Drug | Dosage (mg/kg of weight) | | Thrombolytic Activity (%) | Amount of Residual Fibrinogen (%) |
|---|---|---|---|---|
| Control (physio-logical saline) | | (5)[a] | 34 ± 5 | 117 ± 5 |
| ProUK | 0.5 | (4) | 48 ± 12 | 103 ± 9 |
| | 1.0 | (5) | 54 ± 7 | 101 ± 8 |
| | 2.0 | (7) | 78 ± 7 | 86 ± 4 |
| | 3.0 | (5) | 91 ± 2 | 38 ± 14 |
| | 4.0 | (6) | 92 ± 3 | 34 ± 18 |
| ProUK/FU 1-74 whole IgG molecule | 0.031 | (5) | 35 ± 3 | 99 ± 4 |
| | 0.063 | (4) | 66 ± 7 | 94 ± 17 |
| | 0.125 | (5) | 85 ± 6 | 101 ± 3 |
| | 0.25 | (5) | 85 ± 8 | 88 ± 31 |
| | 0.5 | (5) | 92 ± 3 | 84 ± 5 |
| | 1.0 | (4) | 97 ± 0 | 22 ± 10 |
| | 2.0 | (6) | 91 ± 3 | 3 ± 3 |
| ProUK/FU 1-74 F(ab')$_2$ | 0.031 | (5) | 39 ± 8 | 102 ± 5 |
| | 0.063 | (4) | 78 ± 1 | 99 ± 6 |
| | 0.125 | (4) | 88 ± 4 | 94 ± 10 |
| | 0.25 | (6) | 73 ± 13 | 90 ± 3 |
| | 0.5 | (5) | 89 ± 8 | 102 ± 10 |
| | 1.0 | (5) | 79 ± 12 | 92 ± 2 |
| | 1.5 | (4) | 80 ± 11 | 87 ± 3 |
| | 2.6 | (3) | 96 ± 1 | 26 ± 14 |

a) The figures in parentheses indicate the number of experimental animals.

| Given Drug | Dosage (mg/kg of weight) | | Amount of Residual $\alpha_2$-Antiplasmin (%) |
|---|---|---|---|
| Control (physiological saline) | | (5) | $90 \pm 22$ |
| ProUK | 0.5 | (4) | $65 \pm 6$ |
| | 1.0 | (5) | $81 \pm 9$ |
| | 2.0 | (7) | $76 \pm 8$ |
| | 3.0 | (5) | $52 \pm 18$ |
| | 4.0 | (6) | $22 \pm 12$ |
| ProUK/FU 1-74 whole IgG molecule | 0.031 | (5) | $59 \pm 22$ |
| | 0.063 | (4) | $116 \pm 19$ |
| | 0.125 | (5) | $85 \pm 11$ |
| | 0.25 | (5) | $61 \pm 13$ |
| | 0.5 | (5) | $76 \pm 25$ |
| | 1.0 | (4) | $14 \pm 6$ |
| | 2.0 | (6) | $0 \pm 0$ |
| ProUK/FU 1-74 F(ab')$_2$ | 0.031 | (5) | 115 |
| | 0.063 | (4) | 97 |
| | 0.125 | (4) | $110 \pm 3$ |
| | 0.25 | (6) | $112 \pm 12$ |
| | 0.5 | (5) | $94 \pm 10$ |
| | 1.0 | (5) | $74 \pm 17$ |
| | 1.5 | (4) | $73 \pm 8$ |
| | 2.6 | (3) | 0 |

Addition of bispecific antibody FU 1-74 (whole IgG molecules) (○) described in Reference Example 16 and the F(ab')$_2$ fraction (△) described in this example enhanced the thrombolytic activity of ProUK (●) 18 times and 23 times, respectively.

On the other hand, the amount of decomposed fibrinogen and the amount of consumed $\alpha_2$-antiplasmin were measured as indications of side effects for comparison at a dosage necessary to induce 50% thrombolytic activity. When the whole IgG molecules and the F(ab')$_2$ fraction were used, the amount of decomposed fibrinogen was reduced to one-fifth and one-twentieth, respectively, compared with the case of ProUK alone. The amount of consumed $\alpha_2$-antiplasmin was also reduced to one-third and one-thirteenth, respectively, compared with the case of ProUK alone. When the F(ab')$_2$ fraction was compared with the whole IgG molecules, the thrombolytic activity was somewhat increased about 1.3 times. Conversely, as to the side effects, the amount of decomposed fibrinogen was significantly reduced to one-fourth and the amount of consumed $\alpha_2$-antiplasmin to one-fifth.

Example 3

18

Preparation of F(ab')₂ of Anti-UK-Anti-Human Fibrin Bispecific Hybrid Antibody

(1) Preparation of Monospecific Antibody

Anti-human fibrin specific antibody-producing hybridoma FIB 1-11 described in Reference Example 10 was inoculated intraperitoneally, and treated in accordance with the method described in Example 1-(1) to prepare an IgG fraction of antibody FIB 1-11.

Similarly, using anti-UK-antibody-producing hybridoma UK 1-3 described in Reference Example 12, an antibody IgG fraction produced thereby was prepared.

From the about 50 ml ascites fluids, $IgG_1$ antibodies were obtained in amounts of 180 mg and 230 mg, respectively.

(2) Preparation of F(ab')₂

Two kinds of antibody IgG fractions obtained in (1) were each subjected to the known method [M. Mariani et al., Mol. Immunol., 28, 69 (1991)] to hydrolyze them in Tris-hydrochloric acid buffer (pH 7.0) using ficin (sold by Sigma). L-cysteine (sold by Wako Pure Chemical Industries) was added thereto to a final concentration of 1 mM to activate reaction, followed by incubation at 37°C for 4 hours. After the reaction was terminated by addition of 100 mM N-ethyl maleimide, the resulting antibody fragment solutions were dialyzed against PBS (pH 7.4). Each of the antibody solutions was added to a fibrin-bound column and a UK-bound column to isolate fractions having antigen binding activity, which were further subjected to gel filtration column chromatography to obtain an F(ab')₂ fraction.

(3) Preparation of Heterodimeric F(ab')₂

The antibody FIB 1-11-derived F(ab')₂ fraction obtained in (2) was reduced with DTT, and then the sulfhydryl group of the FIB 1-11 Fab' fraction was maleimidated using o-phenylenedimaleimide according to the known method [M. J. Glennie et al., J. Immunol., 139, 2367 (1987)].

On the other hand, the antibody UK 1-3-derived F(ab')₂ fraction obtained in (2) was similarly reduced with DTT, and then excess DTT was removed by gel filtration chromatography. The resulting fraction was added to the above-mentioned maleimidated FIB 1-11 Fab' fraction in a molar ratio of 1:1, and the antibody Fab' fractions having two kinds of different specificities were bound to each other through a thioether bond in a heterodimeric manner.

The resulting reaction mixture was subjected to gel filtration chromatography using an Ultro-Gel AcA44 column (sold by LKB) to obtain a purified heterodimeric F(ab')₂ fraction.

Example 4

Preparation of F[ab')₂ Fraction of Anti-UK-Anti-Human Fibrin Bispecific Hybrid Antibody

(1) Purification of Monospecific Antibody

Antibody IgG fractions produced by anti-human fibrin specific antibody-producing hybridoma FIB 1-11 described in Reference Example 10 and anti-UK antibody-producing hybridoma UK 1-3 were prepared according to the method described in Example 3-(1).

(2) Purification of (Fab')₂

The two kinds of antibody IgG fractions were each hydrolyzed with pepsin according to a method known in the art. Namely, 18 ml of a 5 mg/ml solution of IgG in 20 mM acetate buffer (pH 3.5) was subjected to the pepsin-coupled column (1X3 cm) described in Example 1-(2). Elution was conducted at 37°C at a flow rate of 3 ml/hour. The effluent was adjusted to pH 7.5, and then subjected to a protein A column (2.6X4.7 cm) equilibrated with PBS to obtain fractions which flowed through without being adsorbed by the column. Then, the resulting fractions were subjected to the fibrin-bound column equilibrated with PBS described in Example 1-(1) to conduct elution with 0.2 M glycine-hydrochloric acid buffer.

From the 90 mg IgG fractions, FIB 1-11 F(ab')₂ and UK 1-3 F(ab')₂ were obtained in amounts of 15 mg and 11 mg, respectively.

(3) Preparation of Fab'-SH

Each of the F(ab')$_2$ antibodies was dissolved in 0.2 M Tris-hydrochloric acid buffer (pH 8.0) supplemented with 10 mM EDTA, and 2-mercaptoethanol was added thereto to a final concentration of 20 mM. After reaction at 30°C for 30 minutes, the reaction product was subjected to a Sephadex G-25 column to conduct elution with 0.1 M sodium phosphate buffer (pH 7.0) supplemented with 0.5 mM EDTA (hereinafter occasionally referred to as PE buffer).

(4) Preparation of Maleimidated Fab'

To the 5 mg/6.7 ml Fab'-SH solution obtained in (3), 0.2 ml of a DMF solution of 0.1 M 1,6-bismaleimido-hexane was added, and reacted at 4°C for 30 minutes. The reaction product was concentrated to 2 ml by ultrafiltration, and then subjected to a Sephadex G-25 column equilibrated with PB buffer.

(5) Preparation of Bispecific F(ab')$_2$ Antibody

A combined solution of a solution of 5 mg of the maleimidated Fab' fraction of the UK 1-3 antibody in PE buffer and a solution of 6.5 mg of the Fab'-SH solution of the FIB 1-11 antibody in PE buffer was concentrated to 3 ml by ultrafiltration, and reacted at 30°C for 30 minutes. 2-Mercaptoethanol was added thereto to a final concentration of 20 mM, and reacted 30°C for 30 minutes. Then, monoiodoacetic acid amide was further added thereto to a final concentration of 25 mM, and allowed to stand at 0°C for 40 minutes. The reaction solution was subjected to a Sephacryl S-200HR column (1.6X100 cm) equilibrated with 0.2 M Tris-hydrochloric acid buffer (pH 8.0) supplemented with 10 mM EDTA. Thus, 5.9 mg of the desired bispecific F(ab')$_2$ antibody was obtained.

(6) Bispecific Antibody Activity

The F(ab')$_2$ fraction obtained in (5), as well as the whole IgG molecules of FU 1-74 described in Example 1-(1), was subjected to the EIA described in Reference Example 7 to measure bispecific antibody activity.

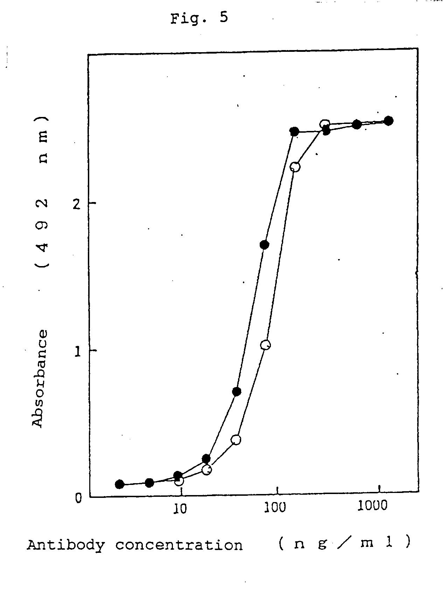

Results thereof are shown in Fig. 5. The F(ab')$_2$ fraction (○) showed bispecific antibody activity comparable to that of the whole IgG molecules (●).

Test Example 3

Enhancement of ProUK Fibrinolysis Activity

For the whole IgG molecule of FU 1-74 obtained in Example 1-(1) and the F(ab')$_2$ fraction obtained in Example 4-(6), the effect of enhancing the thrombolytic activity of ProUK and the function of enhancing the degradation of fibrinogen and $\alpha_2$-antiplasmin were assayed according to the method described in Test Example 2 using the hamster pulmonary embolus model.

Results thereof are shown in Table 2.

## Table 2

| Given Drug | Number of Animals | Dosage (mg/kg of weight) | Thrombo-lytic Activity (%) | Amount of Residual Fibrinogen (%) |
|---|---|---|---|---|
| Control (physio-logical saline) | 1 | - | 25 | 106 |
| ProUK/FU 1-74 whole IgG molecule | 4 | 1.0 | $92 \pm 4$ | $57 \pm 9$ |
| ProUK/FU 1-74F(ab')$_2$ | 4 | 1.0 | $92 \pm 5$ | $96 \pm 5$ |
| (twice) | 4 | 2.0 | $92 \pm 5$ | $66 \pm 11$ |

| Given Drug | Amount of Residual $\alpha_2$-Antiplasmin (%) |
|---|---|
| Control (physio-logical saline) | 86 |
| ProUK/FU 1-74 whole IgG molecules | $10 \pm 4$ |
| ProUK/FU 1-74F(ab')$_2$ | $58 \pm 7$ |
| (twice) | $42 \pm 23$ |

The bispecific antibody F(ab')$_2$ fragment obtained in Example 4 exhibited an effect of enhancing the thrombolytic activity of ProUK comparable to that of the whole IgG molecules of bispecific antibody FU 1-74. On the other hand, the amount of decomposed fibrinogen and the amount of consumed $\alpha_2$-antiplasmin showed significantly low values compared with those of the ProUK/IgG complex, whereby the effect of reducing the side effects was confirmed.

## Example 5

Antigenicity of Anti-UK-Anti-Human Fibrin Bispecific Hybrid Antibody F(ab')$_2$

Thr whole IgG molecule of FU 1-74 (1.5 mg/kg of body weight) described in Example 1-(1) and the F-(ab')$_2$ fragment of FU 1-74 (1.0 mg/kg of body weight) described in Example 1-(2) were given intravenously to the macacas 3 times for each month. The blood was collected for every 10 days during the observation period to assay the antibody titer of monkey anti-mouse FU 1-74. Namely, the collected monkey plasma was added to a microplate sensitized with the whole IgG molecule of FU 1-74, and reacted at room temperature for 2 hours. The plate was thoroughly washed with PBS-Tw, and then HRP-labeled goat anti-human IgG ($\gamma$-chain specific) antibody (cross reactive to monkey IgG, but unreactive to mouse IgG) was added thereto, further followed by reaction at room temperature for 2 hours. Thereafter, enzyme reaction was conducted in the manner described in Reference Example 1.

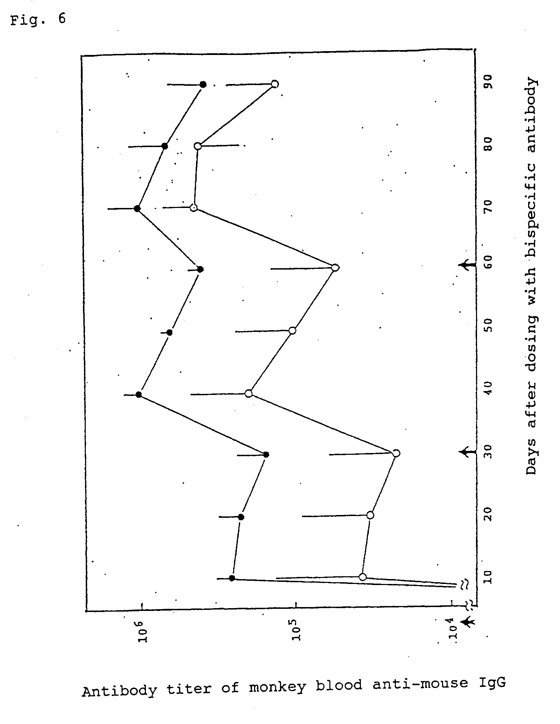

The results are shown in Fig. 6. Both groups (n = 4) which were given the whole IgG bispecific antibody and the F(ab')$_2$ bispecific antibody, respectively, immunologically responded to intravenous administration. However, the F(ab')$_2$-given group (O) showed an antibody titer of one-fifth to one-tenth that of the whole IgG-given group (●).

## Example 6

EP 0 513 778 A2

Preparation of Anti-UK-Anti-Activated platelet Bispecific Hybrid Antibody F(ab')$_2$

(1) Purification of Monospecific antibody

An antibody IgG fraction produced by anti-activated platelet antibody-producing hybridoma 2T60 described in Reference Example 14 was prepared in accordance with the method described in Example 4-(1).

(2) Preparation of Fab'-SH

The antibody IgG fraction obtained in (1) was subjected to the methods described in Example 4-(2) and Example 4-(3) to prepare Fab'-SH.

(3) Preparation of Bispecific F(ab')$_2$ Antibody

A combined solution of a solution of 3.0 mg of the maleimidated Fab' fraction of the UK 1-3 antibody described in Example 4-(4) in PE buffer and a solution of 3.5 mg of the Fab'-SH of the 2T60 antibody prepared in (2) described above in PE buffer was subjected to the method described in Example 4-(5). Thus, 4.8 mg of the desired bispecific F(ab')$_2$ antibody was obtained.

(4) Bispecific Antibody Activity

The F(ab')$_2$ fraction obtained in (3) was subjected to the EIA described in Reference Example 8 to measure bispecific antibody activity.

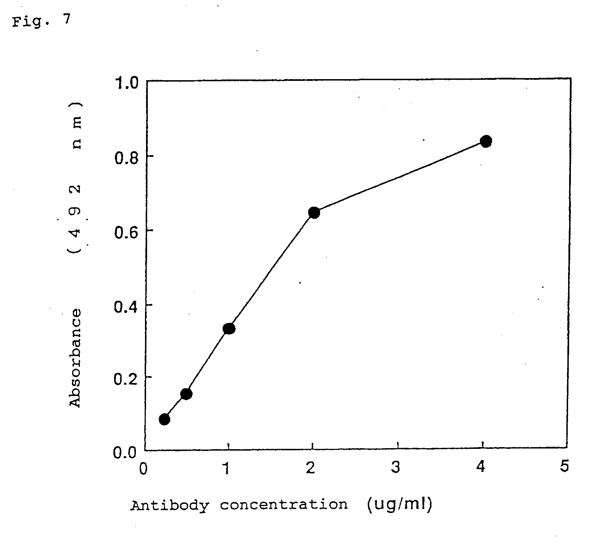

Results thereof are shown in Fig. 7.

## SEQUENCE LISTING

SEQ ID NO:1:

SEQUENCE LENGTH: 12 amino acids

SEQUENCE TYPE: amino acid

TOPOLOGY: linear

MOLECULE TYPE: peptide

FRAGMENT TYPE: N-terminal

SEQUENCE DESCRIPTION: SEQ ID NO:1:

Gly His Arg Pro Leu Asp Lys Lys Arg Glu Glu Cys
1               5                    10

## Claims

1. An antibody comprising an anti-thrombus antibody variable region and anti-thrombolytic substance antibody variable region and having essentially no heavy chain constant region domains 2 and 3.

2. The antibody as claimed in claim 1, wherein said thrombus is fibrin.

3. The antibody as claimed in claim 1, wherein said thrombus is an activated platelet.

22

4. The antibody as claimed in claim 1, wherein said thrombolytic substance is a protease.

5. The antibody as claimed in claim 4, wherein said protease is a plasminogen activator.

6. The antibody as claimed in claim 4, wherein said protease is selected from the group consisting of urokinase, prourokinase, single-chain low molecular weight urokinase, two-chain low molecular weight urokinase and tissue plasminogen activator.

7. The antibody as claimed in claim 4, wherein said protease is prourokinase.

8. The antibody as claimed in claim 1, wherein said antibody is a F(ab')$_2$ fragment.

9. The antibody as claimed in claim 1, wherein said antibody is a F(ab')$_2$ fragment containing an anti-fibrin variable region and an anti-urokinase variable region.

10. A thrombolytic agent comprising the antibody as claimed in claim 1 and a thrombolytic substance immunologically bound thereto.

11. The thrombolytic agent as claimed in claim 10, wherein said thrombolytic substance is protease.

12. The thrombolytic agent as claimed in claim 11, wherein said protease is a plasminogen activator.

13. The thrombolytic agent as claimed in claim 11, wherein said protease is selected from the group consisting of urokinase, prourokinase, single-chain low molecular weight urokinase, two-chain low molecular weight urokinase and tissue plasminogen activator.

14. The thrombolytic agent as claimed in claim 11, wherein said protease is prourokinase.

15. The thrombolytic agent as claimed in claim 10, wherein said antibody containing an anti-fibrin variable region.

16. The thrombolytic agent as claimed in claim 10, wherein said antibody containing an anti-activated platelet variable region.

17. The thrombolytic agent as claimed in claim 10, wherein said antibody is a F(ab')$_2$ fragment.

18. A thrombolytic agent comprising the antibody as claimed in claim 9 and prourokinase immunologically bound thereto.

23

Fig. 1

Fig. 2

Fig. 3

Fig. 4

Dosage of ProUK (mg/kg of weight)

Fig. 5

Fig. 6

Days after dosing with bispecific antibody

Antibody titer of monkey blood anti-mouse IgG

Fig. 7